# EUROPEAN PATENT APPLICATION

(11) **EP 4 616 789 A1**
(43) Date of publication of application: **17.09.2025**
(21) Application number: 24162653.0
(22) Date of filing: 11.03.2024
(51) Int. Cl.: A61B 5/00

(54) **SYSTEM AND METHOD**

(71) Applicant: Comind Technologies Limited, London EC1R 5EJ (GB)
(72) Inventor: PARKER, William, London, EC1R 5EJ (GB); FEER, Suzanna, London, EC1R 5EJ (GB); BEHERA, Anurag, London, EC1R 5EJ (GB)
(74) Representative: Mathys & Squire

(57) **Abstract**

A spectroscopy system comprising: an illumination apparatus configured to direct light towards an object; a light collection apparatus configured to receive light from the object; and a detection apparatus. The light collection apparatus comprises a first fibre and a plurality of second fibres, wherein the first fibre has a larger cross-sectional area than each second fibre. The first fibre is configured to be coupled to the object to receive the light therefrom. The plurality of second fibres are arranged to couple the first fibre to the detection apparatus.

## Description

### Technical Field

The present disclosure relates to the field of systems and methods for the collection of light from an object. For instance, the present disclosure may relate to spectroscopy systems and methods in which a light collection apparatus is used to collect light from an object, using an optical fibre. For example, the present disclosure may be used for interferometric near infrared spectroscopy ('iNIRS') or diffuse correlation spectroscopy (`DCS').

### Background

Spectroscopy methods such as iNIRS and DCS can be used to infer properties of an object by directing light from a light source, such as a laser, towards that object and then using a detector to measure corresponding properties of the light received from that object. The received light may include some of the light which was directed towards the object from the light source, and which subsequently scattered from the object and towards the detector. By monitoring this received light over time, and how the received light signals may change, one or more properties of the object can be inferred. For example, GB2619063 discloses non-invasive intracranial pressure sensing systems and methods which utilise iNIRS for non-invasively determining the intracranial pressure of a subject.

It is desirable to provide improvements to such spectroscopic systems and methods.

### Summary

Aspects of the disclosure are set out in the independent claims and optional features are set out in the dependent claims. Aspects of the disclosure may be provided in conjunction with each other, and features of one aspect may be applied to other aspects.

In an aspect, there is provided a spectroscopy system comprising: an illumination apparatus configured to direct light towards an object; a light collection apparatus configured to receive light from the object; and a detection apparatus. The light collection apparatus comprises a first fibre and a plurality of second fibres, wherein the first fibre has a larger cross-sectional area than each second fibre. The first fibre is configured to be coupled to the object to receive the light therefrom. The plurality of second fibres are arranged to couple the first fibre to the detection apparatus.

Embodiments may enable the spectroscopy system to collect a greater amount of light from the object due to the larger cross-sectional area of the first fibre, while enabling a high spatial resolution to be preserved due to the smaller cross-sectioned second fibres which each receive light from the first fibre. For example, by using a first fibre with a larger cross-sectional area, more light will be collected into that fibre from the object. Ultimately, this may enable higher measurement sensitivities to be achieved due to the increased amount of signal which can be measured. Each of the second fibres may only receive a single mode or only a few modes of light. The light carried by each second fibre may deteriorate less quickly in signal quality as compared to the first fibre (where there may be many more modes of light). Systems of the present disclosure may enable a greater amount of light to be received on the first fibre from the object, while reducing the likelihood of this light deteriorating too much in quality (e.g., with speckles averaging out), as that light is then transmitted onto the second fibres where that is less likely to occur. Additionally, this may enable an object-facing end of the light collection apparatus to be formed from a more robust component, and one which is simpler to attach to the object (as compared to a plurality of smaller fibres).

The first fibre may comprise a multi-mode fibre (`MMF'). Each second fibre may comprise a single-mode fibre (`SMF') or a few-mode fibre (`FMF'). The cross-sectional area of a fibre may comprise a cross-sectional area of the optical channel, e.g., the core, of that fibre. For example, the first fibre may be a fibre with a larger core diameter than any of the second fibres. The first fibre may be configured to receive more optical modes than the second fibres. For example, each second fibre may be configured to carry up to 20 modes (for an FMF). An SMF may be configured to carry one mode. The first fibre may be configured to carry more modes (e.g., an MMF may carry hundreds or thousands, but is not so limited). The first fibre may be arranged to transmit a plurality of different modes to the second fibres. Each second fibre may be configured to receive one (or more) modes from the first fibre. The cross-sectional area of the first and second fibres may be selected so that the first fibre may receive a plurality of different modes of light and these modes of light may be subsequently transmitted onto a plurality of different second fibres. For example, each second fibre may have a cross-sectional area which is sufficiently small relative to the cross-sectional area of the first fibre that a plurality of such second fibres (e.g., SMFs or FMFs) may be coupled to the same first fibre for receiving light therefrom. Likewise, the cross-sectional area of the first fibre may be sufficiently large to be able to accommodate a plurality of second fibres (e.g., SMFs or FMFs), where each such second fibre is arranged to receive light from the first fibre (e.g., to receive one or more modes of light therefrom).

The first fibre may be removably connectable to the plurality of second fibres. For example, each of the plurality of second fibres may be re-usable with subsequent first fibres. The first fibre may comprise a disposable, e.g., single-use, component. The second fibres (and optionally any coupling for coupling the second fibres to a first fibre) may be repeat useable. This may be advantageous in a clinical setting where the object is a human or animal patient, and any patient contacting components need to be discarded after use. In which case, the first fibre (e.g., an MMF) may be discarded after use without needing to discard the second fibres. The first fibre (e.g., an MMF) may extend from an object-facing surface (e.g., at one end of the fibre) to a second fibre-facing surface (e.g., at the other end of the fibre). The second fibres may be coupled to the first fibre at the second fibre-facing surface. Each second fibre may extend from a first fibre-facing surface to a detection apparatus end of the fibre. The first fibre-facing surface of each second fibre may be coupled to the second fibre-facing surface of the first fibre. The second fibre-facing surface of the first fibre may have a larger cross-sectional area than the cross-sectional area of the first fibre-facing surface of each second fibre.

The plurality of second fibres may be coupled to the first fibre to be held in a fixed spatial arrangement with respect to the first fibre. The fixed spatial arrangement may be selected so that each second fibre is coupled to receive light from a respective portion of the first fibre. The fixed spatial arrangement may comprise separating each second fibre from its adjacent second fibre(s) to inhibit any overlap in the optical signal received by each second fibre, e.g., to inhibit one second fibre from receiving at least a portion of the same speckle pattern as a neighbouring (adjacent) second fibre.

The system may comprise a coupling for connecting the first fibre to the plurality of second fibres. The coupling may comprise a mechanical coupling. The coupling may be arranged to receive the first fibre to secure the first fibre to the second fibres, e.g., so that the first fibre is removably connectable to the second fibres. The coupling may comprise a mechanical coupling arranged to removably receive the first fibre and to hold said first fibre in a fixed spatial arrangement with respect to the plurality of second fibres. The coupling may comprise a threaded connection. The coupling may comprise a mechanical fibre connector of type FC/APC, FC/PC, SC/APC, SC/PC, or SMA.

Each second fibre may be coupled to the first fibre via an adhesive and/or a lens. The system may comprise a micro-lens array for coupling the first fibre to the plurality of second fibres. The micro-lens array may be arranged to direct the light from the first fibre into the second fibres. For example, the micro-lens array may be arranged so that light from the first fibre is directed onto each second fibre, e.g., so that each second fibre receives a directed light signal from the first fibre. The coupling may comprise an index-matching material.

The illumination apparatus may be configured to emit coherent light towards the object. The light collection apparatus may be configured to receive coherent light which has scattered from the object (e.g., to receive light signals which were directed towards the object using the illumination apparatus and which scattered therefrom to be collected by the light collection apparatus). The illumination apparatus may be configured to provide wavelength-swept emission of light. For example, the illumination apparatus may be configured to provide wavelength-swept emission of coherent light.

Each second fibre may be spaced apart from adjacent second fibres on a second fibre-facing surface of the first fibre. For example, each second fibre may be arranged to couple to a respective portion of the first fibre (e.g., of the second fibre-facing surface of the first fibre), and each of said respective portions of the first fibre may separated from neighbouring (adjacent) portions which are coupled to other second fibres.

The system may comprise a diffuse correlation spectroscopy, DCS system. The system may comprise an interferometric near-infrared spectroscopy, iNIRS, system.

The system may comprise a plurality of first fibres (e.g., a plurality of MMFs). Each first fibre of the system may couple the object to a plurality of second fibres. Each second fibre may be arranged to couple the first fibre (e.g., the MMF) to an input of the detection apparatus. Each second fibre may be coupled to a respective input of the detection apparatus, e.g., to provide light signals carried by that fibre (and thus from a respective portion of the first fibre) to a respective input of the detection apparatus (e.g., so that the detection apparatus may process said light signals independently from light signals received from other second fibres). A plurality of second fibres, e.g., all of them, may be coupled to the same input of the detection apparatus. The system may be configured to time-multiplex the processing of light signals received from different second fibres.

The system may comprise a physiological measurement system. The object may be a portion of a human or animal body. The detection apparatus may be configured to determine an indication of at least one property of blood flow within the object based on the light received from the plurality of second fibres. The detection apparatus may be configured to derive an autocorrelation function associated with received light signals. For example, the detection apparatus may be configured to derive an autocorrelation function from light signals received from each second fibre, or from a plurality (e.g., set) of second fibres. The detection apparatus may be configured to determine an indication of at least one parameter based on signals received from a plurality of fibres. For example, the detection apparatus may be configured to combine (e.g., average) light signals received from different second fibres. The detection apparatus may be configured to average autocorrelation functions from some, e.g., all, of the second fibres.

In an aspect, there is provided an optical fibre coupling for a spectroscopy system, the coupling comprising: a first fibre receiving portion arranged to receive a first fibre; and one or more second fibre receiving portions, wherein each second fibre receiving portion is arranged to receive one or more second fibres, wherein the first fibre has a larger cross-sectional area than each second fibre. The coupling is arranged to optically couple each second fibre received in a second fibre receiving portion of the coupling to a said first fibre received in the first fibre receiving portion of the coupling to enable light collected by said first fibre to be transmitted to said second fibres. The first fibre may comprise an MMF. The second fibres may comprise SMFs and/or FMFs. The coupling may be of the type disclosed above for optically coupling a first fibre to a plurality of second fibres.

In an aspect, there is provided a spectroscopy method comprising: directing light into an object; collecting light received from the object into a first fibre; and providing light from the first fibre to a plurality of second fibres, wherein the first fibre has a larger cross-sectional area than each second fibre, and wherein each second fibre provides the light received from the first fibre to a detection apparatus. The first fibre may comprise an MMF. The second fibres may comprise SMFs and/or FMFs.

Aspects of the present disclosure may comprise light collection apparatuses of the type disclosed herein. For example, in an aspect there is provided a light collection apparatus comprising: a first fibre (e.g., the first fibre may comprise an MMF); and a plurality of second fibres, wherein the first fibre has a larger cross-sectional area than each second fibre (e.g., the second fibres may comprise SMFs and/or FMFs). The first fibre is configured to be coupled to an object to receive coherent light from said object. Each second fibre is arranged to couple the first fibre to a detection apparatus to provide light received from the first fibre to said detection apparatus.

Aspects of the present disclosure may comprise methods of preparing a light collection apparatus. For example, in an aspect there is provided a method of preparing a light collection apparatus (e.g., for performing a physiological measurement), the method comprising: coupling a first fibre (e.g., an MMF), to an object for receiving light from said object; and coupling a plurality of second fibres (e.g., SMFs and/or FMFs) to said first fibre, wherein the second fibres are coupled to a detection apparatus to provide light received from the first fibre to the detection apparatus.

Aspects of the present disclosure may comprise one or more computer program products comprising computer program instructions configured to program a controller to perform any of the methods disclosed herein.

### Figures

Some examples of the present disclosure will now be described, by way of example only, with reference to the figures, in which:
Fig. 1 is a schematic diagram illustrating an example spectroscopy system.
Fig. 2 is a schematic diagram illustrating an example spectroscopy system.
Fig. 3 is a schematic diagram illustrating an example of coupling between first and second optical fibres.

In the drawings like reference numerals are used to indicate like elements.

### Specific Description

Spectroscopy systems and methods of the present disclosure utilise a light collection apparatus for collecting light from an object, and where that light collection apparatus itself is formed of multiple different optical fibres. For this, a larger optical fibre, in the form of a multi-mode fibre, will be connected to the object. That larger optical fibre is then connected to a plurality of smaller optical fibres, which are each in the form of either a single-mode fibre or a few-mode fibre. Light from the object will be received onto the multi-mode fibre and carried towards the smaller optical fibres, where the light from the multi-mode fibre is provided to those smaller fibres. Those smaller optical fibres then carry that light towards a detector for processing those received light signals. The multi-mode fibre will provide a more reliable connection to the object, as it is bigger, stronger and easier to attach to the object (e.g., as compared to a plurality of smaller fibres). The smaller fibres then receive the light from the multi-mode fibre and can carry this light to the detector, where the received light signals can be either processed independently or collectively.

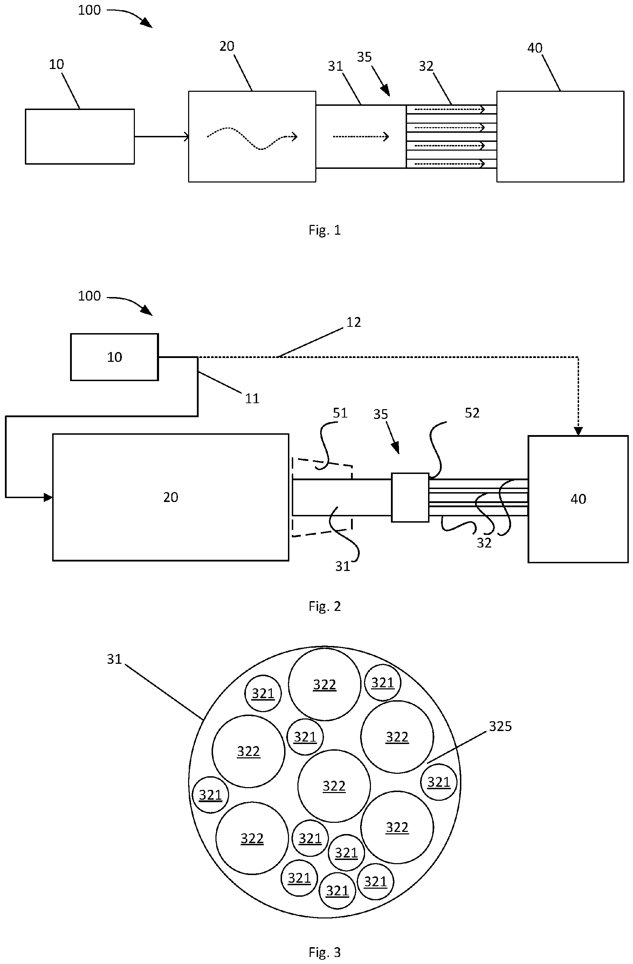

An example spectroscopy system will now be described with reference to Fig. 1.

Fig. 1 shows a spectroscopy system 100. As illustrated in Fig. 1, there is an object 20 to be analysed by the spectroscopy system 100. The spectroscopy system 100 is formed of three groups of different components: (i) an illumination apparatus, (ii) a light collection apparatus, and (iii) a detection apparatus. The dashed arrows in Fig. 1 show the flow of light through the system 100.

The spectroscopy system 100 may be a physiological measurement system. For example, the object 20 may be at least a portion of a human or animal body or living tissue generally, and the system 100 may be for measuring one or more physical properties of that body. The spectroscopy system 100 may comprise a coherent light spectroscopy system. For example, the spectroscopy system 100 may comprise an interferometric near infrared spectroscopy ('iNIRS') system. Alternatively, the spectroscopy system 100 may comprise a diffuse correlation spectroscopy (`DCS') system, such as: (i) a DCS System, (ii) an interferometric DCS ('iDCS') system, or (iii) a time domain DCS ('TD-DCS') system.

The illumination apparatus comprises a light source 10. The light source 10 is coupled to the object 20. The light source 10 may be a coherent light source, such as a laser. For example, the light source 10 may comprise one or more of: (i) a vertical cavity surface emitting laser ('VCSEL'), (ii) a distributed feedback ('DFB') laser, (iii) a distributed Bragg reflector (`DBR') laser, (iv) a laser diode, and/or (v) any other coherent light source. The light source 10 may be coupled to the object 20 by one or more optical fibres. In accordance with various embodiments of the present disclosure, the light source may also be non-coherent.

The object 20 may comprise any object for which non-invasive imaging of that object could be useful. For example, the object 20 may comprise at least a portion of a human or animal body, such as their head/brain. As will be appreciated, it is highly desirable to be able to analyse such regions of the body in a non-invasive manner.

The light collection apparatus comprises a first fibre 31 and a plurality of second fibres 32. The first fibre 31 is coupled to the object 20 at an 'object-facing end' of said first fibre 31. The first fibre 31 is also coupled to each of the second fibres 32 at a 'second fibre-facing end' of said first fibre 31. Each second fibre 32 is coupled to the first fibre 31 at a 'first fibre-facing end' of said second fibre. A coupling 35 is shown in Fig. 1 where the second fibres 32 are coupled to the first fibre 31.

The first fibre 31 has a larger cross-sectional area than any of the second fibres 32. That is, the first fibre 31 is of a larger diameter than any of the second fibres 32. As such, the first fibre 31 may be thicker and more robust than any of the second fibres 32.

In particular, the first fibre 31 comprises a multi-mode fibre (`MMF'). Each second fibre 32 is either a single-mode fibre (`SMF') or a few-mode fibre (`FMF').

As will be appreciated by one of skill in the art, optical fibres may be classified as either single-mode, few-mode or multi-mode based on the number of guided modes they can carry per polarisation direction. An SMF may support only one guided mode per polarisation direction, whereas an MMF may support many. An FMF may support a few modes, but much fewer than an MMF. For example, an FMF may support a number between e.g., 2 and 20 modes, whereas an MMF may support a number which is one or more orders of magnitude higher, such as up to thousands of modes.

An MMF will have a core diameter which is substantially larger than a core diameter of an SMF. For example, an MMF may have a core diameter of 50 micrometres or greater. For an MMF, diameters such as 50, 62.5 or 100 micrometres are typical. Conversely, an SMF may typically have a core diameter in the region of 8 to 10 micrometres (or smaller). An FMF may have a core diameter between the core diameters of typical SMF and MMF, for example 20 micrometres.

Each of the second fibres 32 (the SMFs/FMFs) is coupled to the first fibre 31 (the MMF). As such, the MMF is coupled to a plurality of SMFs and/or FMFs. The MMF therefore couples the object 20 to a plurality of different optical fibres (SMFs/FMFs). The second fibres 32 may be arranged in an array. The array of second fibres 32 is coupled to the MMF. The core of the MMF may be coupled to direct light to the core of each of a plurality of second fibres 32.

The coupling 35 shown in Fig. 1 is the region of the system 100 where the second fibres 32 are coupled to the MMF. The coupling 35 connects the core of the MMF to the core of each second fibre. The coupling 35 may comprise one or more lenses and/or an index-matching material. For example, the coupling 35 may comprise an adhesive between the MMF and the plurality of second fibres 32. Additionally, or alternatively, the coupling 35 may comprise a mechanical element configured to receive the plurality of second fibres 32 and the MMF and to hold the fibres in a fixed spatial arrangement with respect to each other. For example, the coupling 35 may comprise a mechanical fibre connector, such as an FC/APC connector, an FC/PC connector, an SC/APC connector, an SC/PC connector or an SMA connector.

The detection apparatus comprises a detector 40. The detector 40 is coupled to the plurality of second fibres 32. Each second fibre 32 is coupled to the detector 40 at a 'detector-facing end' of said second fibre. Each second fibre 32 couples the MMF to the detector 40. Each second fibre 32 may be connected to a respective input of the detector 40. In some instances, a plurality of detectors 40 may be used, each received a plurality of second fibres. Each second fibre 32 may be coupled to a respective portion of the MMF. For example, the area of the core of the MMF (at the second fibre-facing end) may comprise a plurality of different sub-areas, each of which is connected to a respective corresponding second fibre. Some portions of the area of the core of the MMF may not be coupled to a second fibre.

The spectroscopy system 100 may be configured to analyse the object 20 based on the light received from the object 20 at the detector 40. The system 100 may be configured to detect one or more properties of the received light and/or how the received light signal(s) evolve over time using the detection apparatus.

The illumination apparatus is configured to direct light towards the object 20. The light source 10 is configured to generate light, e.g. coherent light, to be used for analysing the object 20. The illumination apparatus is configured to direct the generated light from the light source 10 towards the object 20, e.g. so that the light may penetrate the surface of the object 20 and scatter within the object. The illumination apparatus may be configured to provide wavelength swept emission of light. For example, the illumination apparatus may be configured to provide emission of coherent light where the wavelength of that light changes over time.

The light collection apparatus is configured to collect light from the object 20. In particular, the light collection apparatus may be arranged to couple to the object 20 to receive light emitted from the illumination apparatus which has scattered from the object 20.

The MMF is configured to be coupled to the object 20. The MMF may be coupled to the object 20 by itself or by an additional component, such as a probe. In some embodiments, the MMF may be considered the probe. The MMF may be coupled to the object 20 by one or more optical elements, e.g., such as a lens or other element for focussing and/or directing light onto the MMF. The MMF is configured to collect the light received from the object 20 and to direct that light away towards the detection apparatus. The coupling 35 is configured to optically couple the MMF to each of the second fibres 32. The light collection apparatus is configured so that light received from the object 20 will travel along the MMF before being transmitted into the second fibres 32. The coupling 35 is arranged to provide an optical path from the MMF to each of the second fibres 32.

Each of the second fibres 32 is arranged to receive light from a respective sub-region of the MMF. Each second fibre 32 may be coupled to the MMF receive to receive a mode of light therefrom. For example, at the second fibre-facing end of the MMF, each second fibre 32 is coupled to a respective sub-region of the MMF for receiving light (e.g., a light mode) therefrom. Each sub-region of the MMF which is coupled to a second fibre 32 may be separated from adjacent sub-regions of the MMF. The sub-regions may be separated by a threshold amount, e.g. to inhibit an individual speckle on the MMF being transmitted to more than one second fibre.

The coupling 35 may be arranged to hold the second fibres 32 in a fixed spatial arrangement with respect to the MMF. The coupling 35 may be configured to secure the MMF to the plurality of second fibres 32 so that the MMF being coupled to the object 20 may optically couple the object 20 to the second fibres 32 via the MMF. The coupling 35 may be configured to removably couple the second fibres 32 to the MMF. For example, the coupling 35 may comprise a plurality of second fibre 32 receiving portions and an MMF receiving portion. Each fibre may be inserted into its respective portion to hold the fibre in place. The fibres may be removably insertable into said portions, e.g. the coupling 35 may comprise a threaded connection. For example, the MMF may be a disposable component which may be removed from the coupling element to be disposed, e.g. and replaced by a subsequent MMF.

Each of the second fibres 32 may be arranged to provide light to the detection apparatus. For example, the light carried by each second fibre 32 may be provided to a respective input of the detection apparatus for processing that light signal. The detection apparatus may be configured to identify one or more properties of the received light signal and to perform analysis of the object 20 based on said identified properties.

An example operation of the spectroscopy system 100 to analyse the object 20 will now be described.

To prepare the spectroscopy system 100 for analysing the object 20, both the illumination apparatus and the light collection apparatus are coupled to the object 20. For the illumination apparatus, one or more optical fibres may be coupled to a surface of the object 20 to provide an optical path from the light source 10 to the surface of the object 20. The optical fibres may be coupled to the object 20 directly or indirectly, e.g. they may themselves be attached to the object 20 or they may be attached via a separate component such as a probe. For the light collection apparatus, the first fibre 31 (the MMF) is coupled to a surface of the object 20. Again, the first fibre 31 may be coupled to the object 20 directly or indirectly. In the example shown in Fig. 1, the illumination apparatus and light collection apparatus are shown as being coupled to the object 20 on opposite sides, but it will be appreciated in the context of the present disclosure that this arrangement is just shown for illustrative purposes. The light collection apparatus may be coupled to the object 20 at a location much closer to the illumination apparatus. The particular location for the two couplings may be selected based on an intended path for photons travelling through the object 20.

To prepare the light collection apparatus, each of the second fibres 32 (SMFs and/or FMFs) are coupled to the first fibre 31 (the MMF). Once coupled, each second fibre 32 may receive light signals from a respective portion of the MMF. That is, each second fibre 32 may be coupled to receive light from a respective sub-region at the second fibre-facing end of the first fibre 31. Each second fibre 32 is also connected to the detector 40 for delivering light signals thereto.

The first fibre 31 may be a disposable (e.g., single-use) component. For this, the second fibres 32 may be pre-connected to the detector 40. To prepare the system 100, a new first fibre 31 may be coupled to the second fibres 32, and also coupled to the surface of the object 20. Similarly, an optical fibre may be coupled to the surface of the object 20 to connect the object 20 to the light source 10. After use, the first fibre 31 may be discarded. Similarly, so may the fibre used for coupling the light source 10 to the object 20. New fibres may be used for a subsequent object 20, but with the same second fibres 32.

To operate the spectroscopy system 100 to analyse the object 20, light is generated by the light source 10 and delivered to the object 20 (e.g. along an optical fibre). The light source 10 may generate coherent light. Some of the light directed towards the object 20 will penetrate the surface of the object 20 and scatter from matter within the object 20. At least some of this light will scatter along one or more optical paths until that light is collected by the light collection apparatus. The light signals received at the first fibre 31 may contain optical wavefronts which optically interfere with one another to provide speckles. As such, the overall light signal which is received into the first fibre 31 may contain a speckle pattern. That is, there may be a plurality of different speckles in that overall light signal, and where those speckles will follow different optical paths through the MMF. As a result, at the second fibre-facing end of the MMF, the overall light signal may contain a plurality of different speckles which are spatially distributed across the MMF (when viewed in cross-section).

The second fibres 32 may be arranged to receive light signals associated with a single speckle, or alternatively, a few speckles. For this, each second fibre 32 is coupled to a respective sub-region of the MMF. Each sub-region may be selected (e.g. is sufficiently small) so that it may carry a single speckle. The spatial distribution of the sub-regions of the MMF may be selected so that each sub-region is separated from its neighbouring sub-regions by at least a threshold distance. That threshold distance may be sufficiently large that a single speckle may not span across two sub-regions. For example, if there were a single speckle which extended beyond the boundary of one sub-region (where that sub-region is connected to one second fibre 32), the separation between that sub-region and adjacent neighbouring sub-regions (where each of those sub-regions is connected to a respective second fibre 32) is of a size selected so that there would be no or minimal extension of that speckle into any of said adjacent neighbouring sub-regions.

In operation, the second fibres 32 each receive light signals from the first fibre 31. For at least some of the second fibres 32, the light signal received will contain a speckle. That light signal will then be carried along the second fibre. For the second fibres 32, as they are SMFs or FMFs (and are small in diameter relative to the MMF), the light signal being carried will contain the one speckle. Conversely, the MMF may carry a plurality of different speckles. As such, some of the second fibres 32 may provide a single (and isolated) speckle to the detector 40, for example. The detector 40 may perform analysis based on each of these individual speckles.

By using this approach, each individual second fibre 32 does not need to be coupled to the object 20. In turn, the coupling of the light collection apparatus to the object 20 may be greatly simplified. Also, the first fibre 31 (the MMF) is much larger and more robust. This may provide an improved reliability for the structural coupling of the fibre to the object 20, as compared to coupling many smaller second fibres 32 to the object 20. Additionally, by using a single MMF as the first fibre 31, if this component is to be disposable (e.g. single-use due to contact with an object such as a human or animal body), the disposable componentry may be cheaper, as it will be a single MMF rather than a plurality of SMFs and/or FMFs.

Moreover, by utilising an MMF to collect light from the object 20, the greater core diameter (and thus surface area open for receiving light signals) may enable more light to be collected into the MMF. For instance, the overall light signal travelling through the MMF may contain a plurality of different speckles. Each individual speckle may then be isolatable through the use of the second fibres 32. For instance, by coupling the MMF 31 to the plurality of second fibres 32, each second fibre 32 may potentially provide an optical path for delivering a single speckle to a detector 40. That way, the detector 40 may be arranged to process different speckles individually despite all of those speckles originally being contained within a single MMF.

The length of the first fibre 31 (the MMF) may be selected to inhibit speckle contrast dropping below a threshold level. For instance, speckles in the speckle pattern may being to average out as they travel along the MMF together. However, the present inventors have identified that the speckle pattern may still be suitably maintained by the MMF when travelling over a reasonable distance. For example, the MMF may be up to 5 metres in length. As will be appreciated in the context of the present disclosure, by reducing the length of MMF, the likelihood of the speckle pattern contrast being reduced may be decreased, but a longer length of MMF may have practical benefits when using the system 100. For example, the object 20 may be a human or animal body, and the MMF may be of sufficient length such that the second fibres 32 and detector 40 can be located away from where the body is. It has been identified by the present inventors that, even in scenarios where the length of the MMF may cause some reduction in contrast of the speckle pattern, the increased amount of received light signals into the MMF (due to the greater core diameter of the MMF, as compared to a plurality of smaller fibres) may outweigh the decrease in speckle pattern contrast. The length of the MMF may be selected to inhibit modal dispersion above a threshold level occurring. For example, the length of MMF may be selected so that the time-of-flight (TOF) distribution does not broaden by more than a threshold amount (e.g., due to differences in arrival times of photons), e.g., and cause too much mixing of various photon paths.

Another example of a spectroscopy system 100 will now be described with reference to Fig. 2. As can be seen, the system 100 of Fig. 2 has a number of similarities to that of Fig. 1. For the sake of brevity, like components will not be described again.

In addition to the components described above in relation to Fig. 1, the spectroscopy system 100 of Fig. 2 also shows a sample arm 11, a reference arm 12, a probe 51 and a mechanical coupling 52.

The spectroscopy system 100 of Fig. 2 may be an interferometric spectroscopy system, such as an iNIRS system. For this, the light source 10 is coupled to both the object 20 and the detection apparatus. The sample arm 11 couples the light source 10 to the object 20 and the reference arm 12 couples the light source 10 to the detection apparatus. Each arm may be provided by an optical fibre, such as an MMF. In particular, the sample arm 11 may comprise an MMF. The probe 51 couples the MMF to the object 20. The mechanical coupling 52 may comprise a coupling 35 of the type mentioned above. The mechanical coupling 52 connects the second fibres 32 to the MMF. For example, on one side, the mechanical coupling 52 receives the MMF and on the other side, the mechanical coupling 52 receives the second fibres 32. The mechanical coupling 52 aligns the second fibres 32 with the MMF. The mechanical coupling 52 may hold the fibres in close proximity to each other for light to be transmitted from the MMF to the second fibres 32.

The light source 10 is configured to generate coherent light, for example. The illumination apparatus is arranged to direct said light from the light source 10 to both the object 20 (via the sample arm 11) and the detection apparatus (via the reference arm 12). Some of the light directed to the object 20 from the light source 10 will travel through the object 20 and be collected by the light collection apparatus. This light is then provided to the detection apparatus. The detection apparatus is configured to combine (e.g. interferometrically) this received light ('sample light') with the light received from the reference arm 12 ('reference light') to provide a combined light signal. The detection apparatus is configured to analyse the object 20 based on the combined light signal.

The probe 51 is configured to couple the MMF to the object 20 so that light from the object 20 may be directed onto the optical channel of the MMF. For example, the probe 51 may comprise one or more optical elements, such as a lens and/or a directing/focussing element. The probe 51 may comprise an object contacting surface for securing the probe 51 to the object 20.

The coupling 52 is configured to optically couple the MMF to the second fibres 32. The coupling 52 may be configured to removably couple the MMF to the second fibres 32. For example, the coupling 52 may be configured so that the MMF is removably insertable into the coupling 52. This may comprise a threaded connection. The coupling 52 may enable MMFs to be selectively connected to the second fibres 32. For example, the coupling 52 is configured so that an MMF is insertable into said coupling 52. The coupling 52 is configured to align the inserted MMF with the second fibres 32. The MMF may be subsequently removed from said coupling 52, e.g. once it has been used. Another MMF may then be inserted into the coupling 52 for alignment with the second fibres 32, e.g. for analysing a different object. The coupling 52 may be configured to ensure a selected alignment occurs between the MMF and the second fibres 32. For example, the coupling 52 may be configured to hold the second fibres 32 in a selected fixed spatial arrangement relative to the MMF once the fibres are inserted into the coupling 52.

Spectroscopy systems of the present disclosure may find particular utility for physiological measurement. In particular, spectroscopy systems of the present disclosure may be configured to detect one or more properties relating to blood flow. For example, systems may be configured to determine an indication of intracranial pressure based on properties of blood flow, e.g. using approaches of the type disclosed in GB2619063.

As mentioned above, the MMF may be configured to receive an overall light signal containing a speckle pattern with a plurality of speckles. The system 100 may be configured to detect changes in the speckle patterns over time. Changes in these speckle patterns over time may represent the movement of scatterers within the object 20. When the object 20 to be analysed comprises a portion of a human or animal body, the movement of scatterers may be dominated by the movement of red blood cells in the microvasculature. The system 100 may be configured to analyse the changes over time in the collected light signal and determine corresponding changes in blood flow in the underlying (e.g., cerebral) tissue based on said changes in the observed speckle patterns. For example, the detection apparatus may be configured to determine an autocorrelation function for the received electric field (referred to as 'g₁(τ)', for example). For this, the detection apparatus may be configured to determine an intensity autocorrelation (referred to as 'g₂(τ)', for example). The detection apparatus may be configured to determine (e.g., extract) g₁(τ) based on the determined g₂(τ). The detection apparatus may be configured to determine an indication of the blood flow based on the determined 'g₁(τ)' function, such as by fitting a mathematical model.

In examples mentioned above, the spectroscopy system 100 may comprise a DCS or iNIRS system. For DCS, the optical intensity (brightness) of either a single speckle, or a small number of speckles in the speckle pattern may be measured at high speed (usually in the range of 100 to 500 kHz), and the autocorrelation of the resulting time-series may be used to determine an index of blood flow. The detection apparatus may be configured to determine that the blood flow is moving faster if the measured signal is decorrelating more quickly. For iNIRS, the detection apparatus may be configured to determine the autocorrelation functions based on the combined light signal resulting from interferometrically combining sample and reference light.

The plurality of second fibres 32 in the light collection apparatus are arranged to direct light to the detection apparatus. The system 100 may be arranged so that a light signal carried along a second fibre 32 is processed independently by the detection apparatus. For example, each second fibre 32 may be connected to a respective input of the detection apparatus. The detection apparatus may be configured to process the light signals received at each input to obtain a measurement for the light signals carried by each second fibre. In other words, for an overall light signal carried by the MMF, the detection apparatus may be configured to obtain data from a plurality of different sub-regions of that MMF (i.e. by measuring the light signals carried along each of the second fibres 32). The detection apparatus may be configured to perform averaging across the second fibre signals. That is, the detection apparatus may be configured to analyse the object 20 based on an average of all or some of the measurements performed for the second fibres 32. For example, the detection apparatus may be configured to determine an average autocorrelation function (e.g. a global average) for the MMF by averaging autocorrelation functions determined for each individual second fibre light signal (e.g. by averaging the determined values for the sub-regions of the MMF).

Additionally, or alternatively, the detection apparatus may be configured to analyse individual light signals associated with particular sub-regions of the MMF. The detection apparatus may store an indication of the particular spatial arrangement of the second fibres 32 relative to the MMF. The detection apparatus may be configured to analyse the object 20 based on the received light signals from individual second fibres 32 (e.g. and their relative location with respect to the MMF). Additionally, or alternatively, the detection apparatus may be configured to process light signals from some or all of the second fibres 32 collectively, e.g., to cover a larger portion of the MMF.

An example of an arrangement of second fibres 32 relative to the first fibre 31 is shown in Fig. 3.

Fig. 3 shows second fibres 32 in the form of both SMFs 321 and FMFs 322. As can be seen, the first fibre 31 - the MMF - is much larger than the second fibres 32. As such, a number of the second fibres 32 fit within the cross-sectional area of the first fibre 31. It will be appreciated in the context of the present disclosure that the arrangement shown in Fig. 3 is purely illustrative. For example, the second fibres 32 may be all SMFs 321 or they may be all FMFs 322. The spatial arrangement may be selected to maximise the amount of the cross-sectional area of MMF which is coupled to a second fibre. As shown in Fig. 3, each second fibre 32 may be coupled to a respective sub-region of the MMF. Adjacent sub-regions may be separated from each other by a spacing 325. The spacing 325 may be selected to inhibit one speckle overlapping into two second fibres 32.

The spatial distribution of second fibres 32 may be controlled by a mechanical coupling 52 of the type described above. For example, the mechanical coupling 52 may comprise a body of material which contains second fibre receiving portions (e.g. apertures) for receiving second fibres 32 according to the selected spatial distribution (e.g. with selected spacings 325 therebetween).

It will be appreciated in the context of the present disclosure that the examples described herein should not be considered limiting. Where relevant, the figures are intended to illustrate the technology in a simplified manner to facilitate easier understanding. For example, the spectroscopy system 100 may comprise a plurality of illumination apparatuses and/or light collection apparatuses. Light from one light source 10 may be collected by multiple different light collection apparatuses and/or a light collection apparatus may collect light from a plurality of different light sources. Similarly, the arrangement of the illumination apparatuses and light collection apparatuses relative to the object should not be considered limiting. The particular arrangement (e.g., the spatial arrangement of illumination and collection apparatuses) chosen may be selected based on the object 20 to be analysed and/or a target volume within the object 20 to be analysed. For example, there may be a greater spacing between them when trying to analyse deeper into the object 20.

As described above, the present disclosure relates to a system in which a plurality of second fibres are each coupled to a first fibre for receiving light therefrom. In the examples given, the first fibre is in the form of a multi-mode fibre (`MMF'), and each second fibre is either a single-mode fibre (`SMF') or a few-mode fibre (`FMF'). In such examples, light collected by the MMF is transmitted to an SMF or an FMF for coupling to a detection apparatus. As the cross-sectional area (diameter) of the MMF is much larger than that of either the SMFs or the FMFs, a plurality of such second fibres may be coupled to the MMF to receive light (e.g., a respective mode) therefrom. However, as will be appreciated in the context of the present disclosure, the first fibre may comprise an alternative type of fibre to an MMF while still being able to receive a plurality of modes of light and to provide these to a plurality of second fibres. For example, the first fibre may comprise an FMF. The first fibre FMF may have a large cross-sectional area (core diameter). This FMF may be coupled to a plurality of second fibres (e.g., SMFs or FMFs), where each of those second fibres has a smaller cross-sectional area, e.g., so that the light from that first fibre FMF may be transmitted to a plurality of such second fibres. For example, the first fibre may be an FMF and the second fibres may each be SMFs. In which case, the first fibre FMF may be arranged to provide a respective mode of light to each of the plurality of SMFs.

It will be appreciated from the discussion above that the examples shown in the figures are merely exemplary, and include features which may be generalised, removed or replaced as described herein and as set out in the claims. With reference to the drawings in general, it will be appreciated that schematic functional block diagrams are used to indicate functionality of systems and apparatus described herein. In addition, the processing functionality may also be provided by devices which are supported by an electronic device. It will be appreciated however that the functionality need not be divided in this way, and should not be taken to imply any particular structure of hardware other than that described and claimed below. The function of one or more of the elements shown in the drawings may be further subdivided, and/or distributed throughout apparatus of the disclosure. In some examples the function of one or more elements shown in the drawings may be integrated into a single functional unit.

As will be appreciated by the skilled reader in the context of the present disclosure, each of the examples described herein may be implemented in a variety of different ways. Any feature of any aspects of the disclosure may be combined with any of the other aspects of the disclosure. For example, method aspects may be combined with apparatus aspects, and features described with reference to the operation of particular elements of apparatus may be provided in methods which do not use those particular types of apparatus. In addition, each of the features of each of the examples is intended to be separable from the features which it is described in combination with, unless it is expressly stated that some other feature is essential to its operation. Each of these separable features may of course be combined with any of the other features of the examples in which it is described, or with any of the other features or combination of features of any of the other examples described herein. Furthermore, equivalents and modifications not described above may also be employed without departing from the invention.

Certain features of the methods described herein may be implemented in hardware, and one or more functions of the apparatus may be implemented in method steps. It will also be appreciated in the context of the present disclosure that the methods described herein need not be performed in the order in which they are described, nor necessarily in the order in which they are depicted in the drawings. Accordingly, aspects of the disclosure which are described with reference to products or apparatus are also intended to be implemented as methods and vice versa. The methods described herein may be implemented in computer programs, or in hardware or in any combination thereof. Computer programs include software, middleware, firmware, and any combination thereof. Such programs may be provided as signals or network messages and may be recorded on computer readable media such as tangible computer readable media which may store the computer programs in non-transitory form. Hardware includes computers, handheld devices, programmable processors, general purpose processors, application specific integrated circuits (ASICs), field programmable gate arrays (FPGAs), and arrays of logic gates.

Other examples and variations of the disclosure will be apparent to the skilled addressee in the context of the present disclosure.

## Claims

1. A spectroscopy system comprising:
an illumination apparatus configured to direct light towards an object;
a light collection apparatus configured to receive light from the object; and
a detection apparatus;
wherein the light collection apparatus comprises a first fibre and a plurality of second fibres, wherein the first fibre has a larger cross-sectional area than each second fibre;
wherein the first fibre is configured to be coupled to the object to receive the light therefrom; and
wherein the plurality of second fibres are arranged to couple the first fibre to the detection apparatus.

2. The system of claim 1, wherein the first fibre comprises a multi-mode fibre, MMF, and each second fibre comprises a single-mode fibre, SMF, or a few-mode fibre, FMF.

3. The system of any preceding claim, wherein the first fibre is removably connectable to the plurality of second fibres.

4. The system of any preceding claim, wherein the plurality of second fibres are coupled to the first fibre to be held in a fixed spatial arrangement with respect to the first fibre.

5. The system of any preceding claim, further comprising a coupling for connecting the first fibre to the plurality of second fibres.

6. The system of claim 5, wherein the coupling is a mechanical coupling arranged to removably receive the first fibre and to hold said first fibre in a fixed spatial arrangement with respect to the plurality of second fibres.

7. The system of claim 5 or 6, wherein the coupling comprises a mechanical fibre connector of type FC/APC, FC/PC, SC/APC, SC/PC, or SMA.

8. The system of any preceding claim, wherein each second fibre is coupled to the first fibre via an adhesive and/or a lens, optionally wherein the system comprises a micro-lens array arranged to direct the light from the first fibre into the plurality of second fibres.

9. The system of any preceding claim, wherein the coupling comprises an index-matching material.

10. The system of any preceding claim, wherein at least one of:
the illumination apparatus is configured to emit coherent light towards the object; and
the illumination apparatus is configured to provide wavelength-swept emission of light.

11. The system of any preceding claim, wherein each second fibre is spaced apart from adjacent second fibres on a second fibre-facing surface of the first fibre.

12. The system of any preceding claim, wherein the system comprises a diffuse correlation spectroscopy, DCS, or an interferometric near-infrared spectroscopy, iNIRS, system.

13. The system of any preceding claim, wherein the object is a portion of a human or animal body, and wherein the detection apparatus is configured to determine an indication of at least one property of blood flow within the object based on the light received from the plurality of second fibres.

14. An optical fibre coupling for a spectroscopy system, the coupling comprising:
a first fibre receiving portion arranged to receive a first fibre; and
one or more second fibre receiving portions, wherein each second fibre receiving portion is arranged to receive one or more second fibres, wherein the first fibre has a larger cross-sectional area than each second fibre;
wherein the coupling is arranged to optically couple each second fibre received in a second fibre receiving portion of the coupling to a said first fibre received in the first fibre receiving portion of the coupling to enable light collected by said first fibre to be transmitted to said second fibres.

15. A spectroscopy method comprising:
directing light into an object;
collecting light received from the object into a first fibre; and
providing light from the first fibre to a plurality of second fibres, wherein the first fibre has a larger cross-sectional area than each second fibre, and wherein each second fibre provides the light received from the first fibre to a detection apparatus.
